Europäisches Patentamt

European Patent Office

Office européen des brevets

(11) Publication number: **0 525 212 A1**

## EUROPEAN PATENT APPLICATION
### published in accordance with Art. 158(3) EPC

(21) Application number: **92905301.5**

(22) Date of filing: **19.02.92**

(86) International application number:
**PCT/JP92/00173**

(87) International publication number:
**WO 92/15009 (03.09.92 92/23)**

(51) Int. Cl.5: **G01N 21/76**, G01N 33/536

(30) Priority: **19.02.91 JP 46053/91**

(43) Date of publication of application:
**03.02.93 Bulletin 93/05**

(84) Designated Contracting States:
**CH DE FR GB IT LI NL**

(71) Applicant: **TDK CORPORATION**
**13-1, Nihonbashi 1-chome**
**Chuo-ku, Tokyo 103(JP)**

(72) Inventor: **SHIBUE, Akira 3-22-14-208, Nakacho**
**Koganei-shi**
**Tokyo 184(JP)**
Inventor: **TANAKA, Masaru 2-13-15-203,**
**Saiwaicho**
**Chiba-shi**
**Chiba 260(JP)**
Inventor: **KAMIYA, Shinji 3-38-18-201,**
**Maeharacho**
**Koganei-shi**
**Tokyo 184(JP)**
Inventor: **AIZAWA, Masuo 2-19-14, Amanuma**
**Suginami-ku**
**Tokyo 167(JP)**

(74) Representative: **Votier, Sidney David et al**
**CARPMAELS & RANSFORD 43, Bloomsbury**
**Square**
**London WC1A 2RA(GB)**

(54) **METHOD OF ANALYZING ELECTROCHEMILUMINESCENT SUBSTANCE AND APPARATUS THEREFOR.**

(57) A method of determining an electrochemiluminescent substance with high measurement accuracy. An operation electrode, an opposed electrode and a reference electrode are disposed in a liquid sample containing a substance which causes electrochemiluminescence by electrolytic oxidation in the presence of activated oxygen, and the operation electrode is set to an oxidation potential of the substance in the presence of activating oxygen so as to cause electrochemiluminescence on the operation electrode and to measure the quantity of emission. The present invention is useful in luminescence immunoassay utilizing an antigen-antibody reaction.

Rank Xerox (UK) Business Services

*F I G. 1*

Technical Field

The present invention relates to determination of an electrochemiluminescent substance dissolved in a liquid sample.

Background Art

Electrochemiluminescent substances such as luminol, pyrene, etc. have been utilized as labels for antigens and antibodies in various kinds of immunoassays. Typical methods known for determination of an electrochemiluminescent substance include a method in which a chemiluminescent substance is electrochemically brought into luminescence by a two-electrode technique and the amount of luminescence is measured (Japanese Pre-examination Patent Publication (KOKAI) No. 63-218846, and U.S. Patent No. 4,280,815). In the determination method, a flow cell is used.

The method of determining an electrochemiluminescent substance by the two-electrode technique comprises placing a working electrode and a counter electrode in a liquid sample containing the electrochemiluminescent substance, impressing a voltage between the electrodes to cause electrochemiluminescence at the working electrode functioning as an anode, and measuring the amount of luminescence.

The stronger and stabler the electrochemiluminescence, the higher is the accuracy of measurement which can be achieved. In order to obtain a strong and stable electrochemiluminescence in an aqueous solution in the vicinity of neutrality, the potential of the anode should preferably be set as high as possible within a range such that gaseous oxygen is not evolved through electrolysis of water. In the determination method using the two-electrode technique, however, it is impossible to know the potential of the anode, so that it is very difficult to set the anode potential at an optimal value. For safety, therefore, prevention of the evolution of gaseous oxygen should be made by setting the anode potential at a lower value with a good margin, as compared to the gaseous oxygen evolution potential. As a result, the electrochemiluminescence obtained is weak and the accuracy of measurement is low.

On the other hand, cells of special construction which have been generally used in laboratory work for measurement of electrochemiluminescence require complicated installing operations for keeping proper the positional relationships between a working electrode, a counter electrode and a reference electrode, the arrangement of a liquid sample phase with respect to the electrodes, and the spacing and parallelism between the electrode at which luminescence takes place and an externally disposed optical detector. Besides, flow cells of the two-electrode system, which are commonly used industrially, have the drawback that the potential of a working electrode is not controllable and it is difficult to replace the cell after contamination of electrodes.

Accordingly it is an object of this invention to provide a method of determining an electrochemiluminescent substance with high accuracy, and an apparatus by which the measurement according to the determination method can be carried out through simple operations and which offers a high sensitivity of detection.

Disclosure of the Invention

Thus the present invention provides a method of determining an electrochemiluminescent substance comprising:

placing a working electrode, a counter electrode and a reference electrode in a liquid sample containing an electrochemiluminescent substance capable of chemiluminescence through electrolytic oxidation in the presence of activated oxygen;

setting the working electrode at an oxidation potential for the electrochemiluminescent substance in the presence of activated oxygen to thereby cause electrochemiluminescence at the working electrode; and

measuring the amount of the electrochemiluminescence,

wherein the potential of the working electrode is controlled by use of the reference electrode to below a potential at which gaseous oxygen is generated through electrolysis of water, and also provides a cell for use in the determination method.

According to the method of measuring the electrochemiluminescence of the present invention, it is possible by arranging a reference electrode to control the potential of a working electrode functioning as an anode, to be as high as possible below a potential at which gaseous oxygen is generated through electrolysis of water. With such control, further, it is possible to make the electrochemiluminescent substance luminesce most strongly and, therefore, to obtain a higher accuracy of measurement than that achievable according to the prior art. Besides, the cell for measurement of electrochemiluminescence according to the present invention is suitable for use in the above-described determination method, is detachable, ensures easy operations for positioning, namely, for setting the spacing and parallelism between the cell and an optical detector, and offers a high sensitivity of detection.

## Brief Description of the Drawings

Figure 1 is a vertical sectional view of a cell for measurement of electrochemiluminescence suited to the analytical method according to the present invention.

Figure 2 is a rear view of the cell shown in Figure 1.

Figure 3 is a vertical sectional view of another cell suited to carry out the method of the present invention, in which a thin film of light transmitting electrode functioning as a working electrode is provided at a light transmitting window.

Figure 4 shows a photon counting system used in Examples and Comparative Examples, in which numeral 14 denotes an electrochemiluminescence measuring cell, 15 an optical detector, 16 a preamplifier, 17 a photo-pulse counter, 18 a potentiostat, 19 a function generator, 20 a CPU, and 21 denotes a display device.

Figure 5 shows calibration curves prepared from the results obtained in Examples 1 and 2 and Comparative Example 1.

## Best Mode for Carrying Out the Invention

### Electrochemiluminescent substance

The electrochemiluminescent substance for use in the present invention may be any of those substances which are known to be capable of chemiluminescence through electrolytic oxidation in the presence of activated oxygen. The electrochemiluminescent substances usable include, for example, luminol, luciferin and electrochemiluminescent derivatives thereof such as isoluminol, N-aminohexyl-N-ethylisoluminol, N-aminobutyl-N-ethylisoluminol (ABEI), activated ester derivatives of ABEI, etc., of which preferred is luminol. As has been described above, the electrochemiluminescent substances are oxidized electrochemically on the working electrode functioning at an anode and then chemically react with the aforementioned activated oxygen to thereby luminesce.

### Activated oxygen

The activated oxygen referred to herein includes atomic oxygen, oxygen in the state of constituting hydrogen peroxide, and other forms of oxygen and oxides in activated state which are produced by electrochemical reactions. The activated oxygen may be provided in a liquid sample, for example, by preliminarily adding hydrogen peroxide to the liquid sample, or by reducing oxygen molecules dissolved in the liquid sample or preliminarily added to the liquid sample, through an electrochemical reaction. The hydrogen peroxide is preferably added in such a manner as to obtain a hydrogen peroxide concentration of from 0.03 to 10 mmol/l, more preferably from 0.3 to 3 mmol/l.

### Potential of working electrode

As has been described above, in order to permit an electrochemiluminescent substance to luminesce most strongly, the potential of an anode should be set as high as possible within the range below a potential at which gaseous oxygen is generated through electrolysis of water. According to the method of the present invention, it is possible by use of a reference electrode to monitor the potential of a working electrode serving as an anode, and therefore to control the potential of the working electrode to a desired value.

Specifically, the potential of the working electrode is controlled to be as high as possible in the range below the potential necessary for generation of gaseous oxygen through electrolysis of water. Preferably, the potential of the working electrode is in the range below the gaseous oxygen generation potential and above the theoretical potential window for the electrode, more preferably within 0.2 V below the gaseous oxygen evolution potential.

More specifically, the potential window in the case of using a platinum electrode as a working electrode at pH 7, for example, is said to be around +0.7 to -0.9 V vs. Ag/AgCl. However, the potential necessary for generation of gaseous oxygen through electrolysis of water at pH 7, in the presence of 1 mmol/l of luminol

and 2 mmol/ of hydrogen peroxide in 10 mmol/l(dm$^{-3}$) of a phosphate buffered saline, is about 1.30 V vs. Ag/AgCl. Therefore, according to the method of the present invention, where platinum electrodes are used as a working electrode and a counter electrode, and a silver-silver chloride electrode is used as a reference electrode, the working electrode is set at a potential of lower than 1.3 V, preferably 0.9 V or higher but lower than 1.3 V relative to the reference electrode.

The gaseous oxygen generation potential, in general, varies depending on the materials of the electrodes used, the pH of the aqueous solution to be determined, the concentration of activated oxygen in the solution, and so on. Once these conditions are determined, the gas generation potential under the conditions is easy to know.

In carrying out the method of the present invention, the pH of the liquid sample is set ordinarily in the range of from 5 to 9. If the pH is excessively low, the amount of electrochemiluminescence is reduced. On the other hand, too high values of pH result in that luminescence can occur even if the working electrode is not set at the oxidation potential; in such a situation, the merit of electrochemiluminescence that a limited reaction is permitted to proceed in a controlled period of time cannot be fully displayed, and, as a result, the accuracy of determination is lowered.

The reference electrode for use in the determination method of the present invention may be any of those generally used in three-electrode techniques. For example, hydrogen electrode, silver-silver chloride electrode, calomel electrode, etc. can be used. Among these electrodes, preferred is silver-silver chloride electrode.

Control of the potential of the anode by use of the reference electrode is preferably carried out according to controlled potential electrolysis with the use of a potentiostat.

Apparatus for measurement of electrochemiluminescence

In carrying out the method according to the present invention, a variety of apparatuses suited to carry out the three-electrode technique can be used, without any special limitation. However, particularly preferable apparatuses are provided according to the present invention, as will be described below.

As a first aspect of the apparatus according to the present invention, there is provided an apparatus for measurement of electrochemiluminescence comprising a vessel having two plates disposed opposite to each other to constitute vessel walls, a working electrode, a counter electrode, and a reference electrode, said working, counter and reference electrodes provided at an inner surface of one of said plates, and the other of said plates being light transmissive at least at its portion opposed to said working electrode.

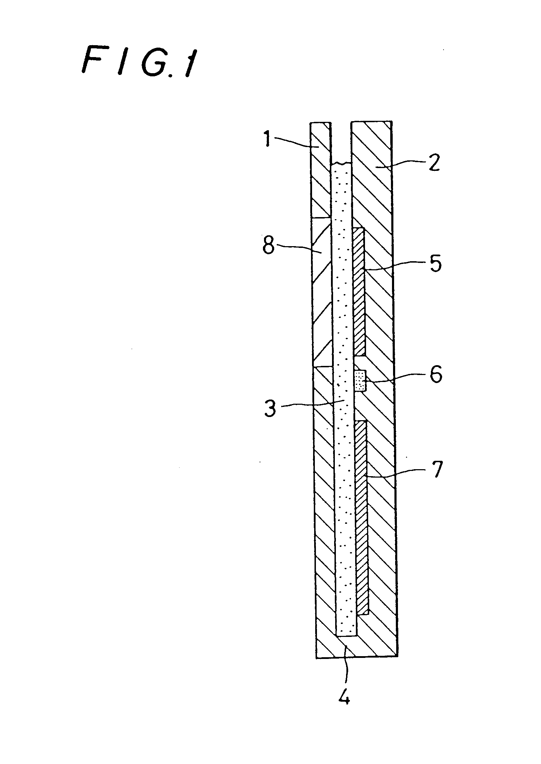

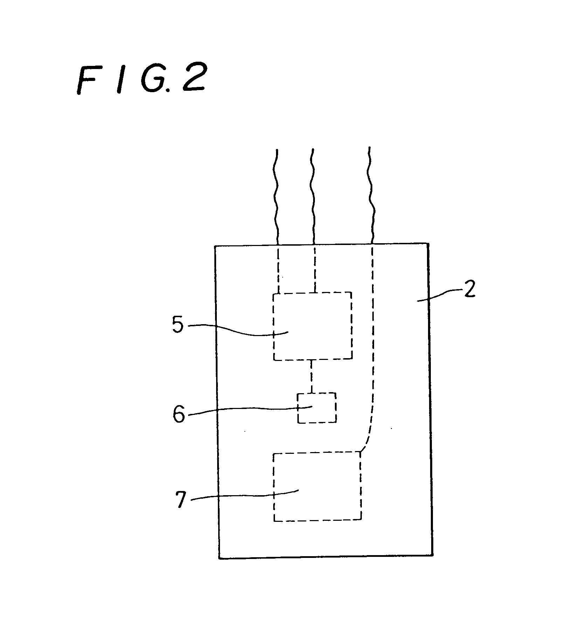

A cell for measurement of electrochemiluminescence, which is one embodiment of the apparatus according to the first aspect, is shown in Figures 1 and 2. Figure 1 shows schematically a vertical section of the cell, and Figure 2 shows a rear view of the cell. Two plates 1 and 2 for forming vessel walls are disposed in parallel and opposite to each other, with an interior space 3 therebetween for accommodating a liquid sample. The plates 1 and 2 and are sealed at their bottom 4 and left and right edges (not shown) to form a vessel having the interior space 3, which has a flat overall shape. As seen from Figures 1 and 2, a working electrode 5, a reference electrode 6 and a counter electrode 7 are disposed at the inner surface of one of the cell walls, 2, so as to front on the interior space 3. On the other hand, the other cell wall 1 is provided with a light transmitting window 8 formed of a light transmitting material at a position opposed to the working electrode 5.

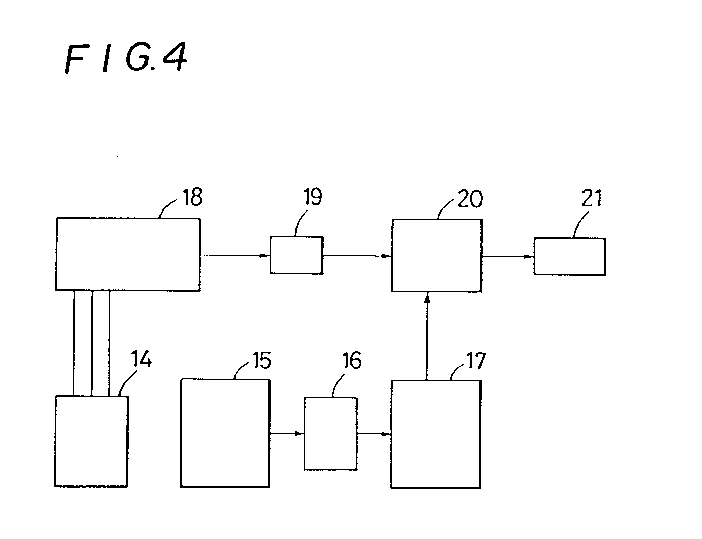

An aqueous solution containing an electrochemiluminescent substance is placed in the interior space 3, and luminescence takes place at the interface between the solution and the working electrode 5 functioning as an anode. Photons generated at the time of the luminescence pass through the light transmitting window 8, to be detected by a photodetector 15 (See Figure 4).

Materials which can be used for forming the cell walls 1 and 2 constituting the cell include, for example, acrylic resins, silicone resins, epoxy resins, styrene resins, vinyl chloride resins, fluororesins, etc.

The light transmitting material constituting the light transmitting window 8 includes, for example, various glasses, acrylic resins such as polymethacrylate, etc. Among the light transmitting materials, one which has a high transmittance at the wavelength of light emitted from the electrochemiluminescent substance used is selected suitably.

The working electrode 5 includes, for example, electrodes comprising a precious metal such as platinum, gold, palladium, etc., carbon such as glassy carbon, graphite, etc., a transparent electrical conductor such as ITO, and so on. The shape of such electrode may be, for example, plate-like, film-like, mesh or screen-like, slit, comb-like, etc.

The counter electrode 7 may ordinarily be any electrode that is satisfactory in regard of material and shape for use as a counter electrode for setting the potential of the working electrode in the aqueous solution used. In general, metallic electrodes comprising a precious metal such as platinum, gold, palladium, etc., silver, nickel, etc., carbon electrodes comprising glassy carbon, graphite, etc. are representative of the usable electrodes. Electrodes comprising a conductive oxide such as $SnO_2$, ITO, etc. can also be used. Further, a counter electrode capable of functioning also as a reference electrode, for example, a flat plate form Ag-AgCl electrode having a projection area of at least three times that of the working electrode can also be used.

The reference electrode 6 includes, for example, hydrogen electrode, silver-silver chloride electrode, calomel electrode, etc., of which preferred is silver-silver chloride electrode.

According to a second aspect of the present invention, there is provided another apparatus for measurement of electrochemiluminescence suited to carry out the method of the present invention, namely, an apparatus for measurement of electrochemiluminescence comprising a vessel having two plates disposed opposite to each other to constitute vessel walls, a working electrode, a reference electrode, and a counter electrode comprising a light transmitting material, said working and reference electrodes provided at an inner surface of one of said plates, and said counter electrode provided at least at that portion of the other of said plates which is opposed to said working electrode.

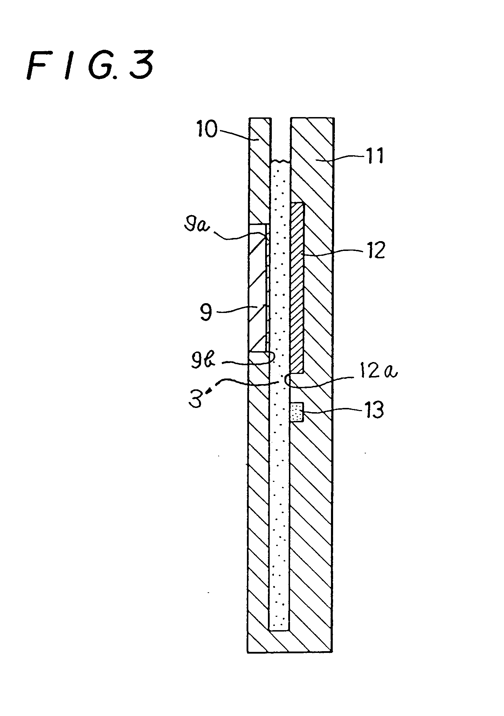

Figure 3 shows a vertical section of an embodiment of the apparatus according to the aforesaid second aspect. The cell as shown in Figure 3 is substantially the same in construction as the cell of Figure 1, except that a thin film of a light transmitting electrode 9a functioning as a working electrode is provided at the inner surface of a light transmitting window 9 formed as a part of a vessel wall 10 and a counter electrode 12 and a reference electrode 13 are provided at the other cell wall 11. Materials for forming the light transmitting electrode 9a include, for example, highly transparent and electrically conductive materials such as ITO, $SnO_2$, etc. Generally, where the working electrode consisting of the light transmitting electrode is thus disposed in close contact with the inner surface of the light transmitting window, a further improved accuracy of measurement is obtained.

In the cell shown in Figure 3, the distance from the working electrode 9a functioning as an anode to a photodetector 15 (See Figure 4) disposed outside the light transmitting window 9 can be set shorter than that in the case of the cell shown in Figure 1. Besides, photons reach the photodetector 15 without passing through the sample 3' containing an electrochemiluminescent substance. Therefore, there is the advantage that the photons are not refracted at the interface between the liquid sample 3' and the light transmitting window 9, and the accuracy of measurement is further improved.

It has been found that, in the apparatus according to the above-described second aspect, the following improvements are effective in stabilizing electrochemiluminescence.

(1) Electrical connection between an ITO electrode and a lead wire therefor is formed by bringing a contact material having a work function of 4.5 eV or below into contact with the ITO electrode. The contact materials usable for the purpose include, for example, tin, lead, silver, iron, chromium, alloys thereof and so on, of which preferred is a 40 wt.% tin-60 wt.% lead solder (work function: 4.3).

Conventionally, the connection between the ITO electrode and the lead wire therefor has been formed by bringing a copper contact material into contact with an ITO film. According to the conventional method, however, a Schottky barrier is formed to increase contact resistance, causing a lowered luminescence and variations in the amount of luminescence. This problem is improved by use of the above-described contact material having a work function of 4.5 eV or below, preferably 4.4 or below.

(2) The lead wire to be connected to the working electrode is separated into a potential-measuring lead wire and a voltage-applying lead wire, which are connected to the ITO electrode by respective contacts. Two contacts are formed.

Heretofore, a single contact has been formed, and a potential-measuring lead wire and a voltage-applying lead wire have been connected to the single contact, with the result of unstable measurement of potentials. The problem is improved by the above-mentioned two-contact system.

(3) The effective area of the working electrode is equal to or smaller than the area of the opposed counter electrode. Preferably, the areas are equal. Evening of the distribution of current density on the working electrode is considered to contribute to stability of luminescence.

(4) The reference electrode is set at a position which is on the outside of a straight line connecting between an end of the working electrode and an end on the same side of the counter electrode disposed in parallel and which is spaced from the straight line by two times (2L) the spacing (L) between the working and counter electrodes.

Although any one of the above improvements (1) to (4) is effective in stabilizing the luminescence, it is desirable to use two or more of the improvements in combination. Use of all of the improvements (1) to (4) together is most preferable.

In the cells shown respectively in Figures 1 and 3, a liquid sample inlet port (not shown) is preferably provided with a guide for pouring a portion of a liquid sample into the cell. With such guide, the liquid sample can be poured into the cell smoothly, without spilling. It is also preferable to provide the cell with a vent hole, at other position than the position of the inlet port; with such vent hole, the liquid sample can be smoothly poured into the cell, because air inside the cell is expelled as the liquid sample is poured into the cell. Furthermore, where supports (not shown) for the electrodes 5, 6, 7, 9, 12 and 13 in the cells shown in Figures 1 and 3 are detachable, it is easy to wash the electrodes, which is convenient for repeated use of the electrodes. Besides, the cell according to the present invention as shown in Figures 1 and 2 may be used as a disposable type cell.

Application

The electrochemiluminescent substance to be analyzed by the method according to the present invention may include those electrochemiluminescent substances which are used as labels in various analyses. One example of such application is luminescence immunoassay in which an antigen-antibody reaction is utilized.

To analyze an immunoreactant (antigen or antibody) in a sample by the luminescence immunoassay, electrochemiluminescent substance is attached as a label to a complementary immunoreactant (antibody or antigen) capable of specifically binding to said immunoreactant in the sample. The antigen can be any of those which have been known, such as various proteins, polypeptides, polysaccharides, lipids, nucleic acids, etc. Besides, the labelling of the immunoreactant with the electrochemiluminescent substance can be carried out by known methods. The labelled complementary immunoreactant obtained in this manner is, for example, added to the sample, and reaction with said immunoreactant in the sample is allowed to take place in an aqueous solution containing an appropriate buffer solution. After the reaction is over, the electrochemiluminescent substance attached to the product of the antigen-antibody reaction is determined by use of the method according to the present invention.

Example 1

A cell as shown in Figure 1 was used as an apparatus for measurement of electrochemiluminescence. A platinum electrode having a thickness of 0.3 mm and an area of 0.32 $cm^2$ was used as a working electrode 5, and a platinum electrode with a thickness of 0.3 mm and an area of 0.60 $cm^2$ was used as a counter electrode 7. As a reference electrode, a silver-silver chloride electrode was used which had a silver wire (0.5 mm$\phi$) as a base, with silver chloride deposited on a 15 mm length portion at one end thereof by an electrolytic technique.

A light transmitting vessel wall 1 was formed of a 1-mm thick plate of polymethyl methacrylate. The vessel wall had a light transmittance of 90% or above at a wavelength of 425 nm. A vessel wall 2 was formed of a 2-mm thick plate of polymethyl methacrylate, and a bottom 4 and side walls are also formed of polymethyl methacrylate. The spacing between the vessel walls 1 and 2 was 1 mm, and the internal volume of the cell was 500 $\mu$l.

The cell was connected into a circuit of a photon counting system, as shown in Figure 4. Into a phosphate buffer saline (PBS) having the composition of 10 mM of $Na_2HPO_4$, 10 mM of $NaH_2PO_4$, 120 mM of NaCl and 2.7 mM of KCl, hydrogen peroxide was dissolved in a concentration of $10^{-3}$ mol/liter, to obtain a base solution. Into the base solution, luminol was dissolved in concentrations of $10^{-13}$, $10^{-12}$, $10^{-11}$, $10^{-10}$, $10^{-9}$, $10^{-8}$, $10^{-7}$, $10^{-6}$ and $10^{-5}$ mol/liter to prepare 9 samples to be tested. Each of the samples was placed in the measuring cell, and a voltage so controlled as to set the potential of the working electrode at 1.2 V in relation to the potential of the Ag-AgCl reference electrode was applied by a potential generator to the working electrode for 15 seconds, thereby permitting the luminol dissolved in the sample to luminesce. The amount of luminescence was measured by use of a photomultiplier as an optical detector 15.

Example 2

The amount of luminescence of luminol was measured in the same manner as in Example 1, except that a voltage so controlled as to set the potential of the working electrode at 0.7 V in relation to the potential of the reference electrode was impressed on the working electrode.

Comparative Example 1

The amount of luminescence of luminol was measured in the same manner as in Example 1, except that measurement was made according to a two-electrode technique by not connecting the reference electrode to the circuit and a voltage so controlled as to set the potential of the working electrode at 1700 mV in relation to the potential of the counter electrode was impressed on the working electrode.

Comparative Example 2

The amount of luminescence of luminol was measured in the same manner as in Example 1, except that a voltage so controlled as to set the potential of the working electrode at 2000 mV in relation to the potential of the Ag-AgCl reference electrode was impressed on the working electrode.

Calibration curves were prepared from the results of the above Examples 1 and 2 and Comparative Example 1. In Comparative Example 2, electrolysis of water caused vigorous evolution of gaseous oxygen at the working electrode and hydrogen gas at the counter electrode, and bubbles of the evolved gases hindered the contact between the electrode surfaces and the solution, thereby rendering it impossible to make measurement and, hence, to prepare a calibration curve.

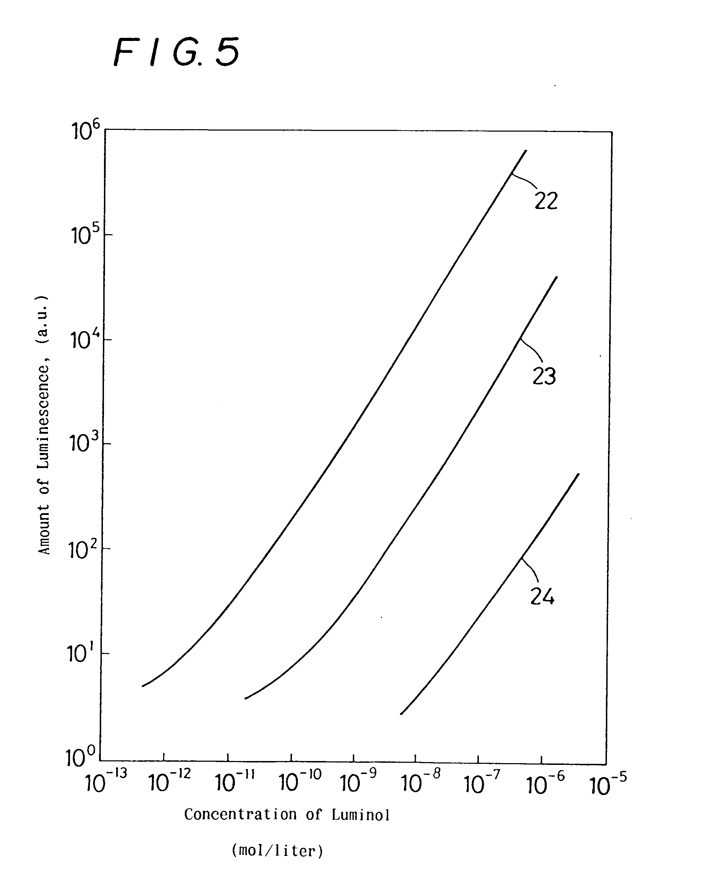

The calibration curves obtained are shown in Figure 5, in which curves 22, 23 and 24 are the calibration curves of Examples 1 and 2 and Comparative Example 1, respectively.

Example 3

A cell as shown in Figure 3 was used as an apparatus for measurement of electrochemiluminescence. The cell is a vessel internally measuring 16 mm (width) x 32 mm (depth) x 1 mm and having an internal volume of 500 $\mu$l, in which a 1-mm thick glass plate 10 and a 2-mm thick polymethyl methacrylate plate 11 are disposed opposite to each other, with a spacing of 1 mm therebetween, and are sealed with polymethyl methacrylate at a bottom and both side portions thereof. A portion of the plate 10 is made of polymethacrylate to constitute a light transmitting window measuring 12 mm x 10 mm, and an ITO thin film electrode 9a with an area of 1.44 cm$^2$ is provided on the inner surface of the window. A platinum electrode with a thickness of 0.3 mm and an area of 0.60 cm$^2$ is provided as a counter electrode 12. As a reference electrode, a silver-silver chloride electrode was used which had a silver wire (0.5 mm$\phi$) as a base, with silver chloride deposited on a 15 mm length portion at one end thereof by an electrolytic technique. Five kinds of cells respectively fulfilling one of the following conditions (A) to (E), in addition to the above-described construction, were used to carry out measurement in the manner described below.

(A): Connection between the ITO electrode lead wire and the ITO electrode was formed by bringing a copper contact material into contact with the ITO electrode, and a voltage-applying lead wire and a potential-measuring lead wire were connected to the contact. The reference electrode 13 was disposed at a distance of 2 mm from the middle point of the line connecting between the end 9b of the working electrode 9a and the end 12a of the counter electrode 12.

(B): The same as in (A) except that a contact comprising copper coated with a solder having a work function of 4.3 eV was used in place of the above copper contact material.

(C): Connection between the ITO electrode lead wire and the ITO electrode was formed by bringing two pieces of a contact material, which comprises copper coated with a solder of a work function of 4.3 eV, into contact with the ITO electrode. A voltage-applying lead wire was connected to one of the contacts, and a potential-measuring lead wire to the other contact.

(D): Contact conditions were the same as in (B), and the reference electrode 13 was disposed at a distance of 4 mm from the middle point of the line connecting the end 9b of the working electrode 9a to the end 12a of the counter electrode 12.

(E): Contact conditions were the same as in (B), and the light transmitting window 9 was replaced by a colored glass filter (tradename: L-40, produced by Irie Seisakusho K.K. ) impervious to light of a wavelength of 400 nm or below.

Hydrogen peroxide was dissolved in PBS in a concentration of $10^{-3}$ mol/liter to form a solution, into which luminol was dissolved in a concentration of $10^{-10}$ mol/liter to prepare a sample. The sample was placed in the cell, and the cell was connected to the photon counting system shown in Figure 4. A voltage so controlled as to set the potential of the working electrode 9a at 0.8 V in relation to the potential of the reference electrode 13 was applied by the potential generator to the working electrode for 15 seconds, thereby causing the luminol dissolved in the sample to luminesce. The amount of luminescence was measured, and a C.V. value was obtained therefrom. The results are shown in Table 1.

Table 1

| Condition | C.V. value |
|-----------|------------|
| (A) | 15.7 |
| (B) | 8.5 |
| (C) | 5.4 |
| (D) | 5.3 |
| (E) | 5.3 |

Industrial Applicability

It is known that electrochemiluminescent substances are utilized as labels in various analyses. The electrochemiluminescent substance to be analyzed by the method of the present invention may be one which is in use as label of this type. Therefore, the method of determining an electrochemiluminescent substance according to the present invention is applicable, for example, to luminescence immunoassays, determination of the activity of complements and applications thereof, research of the process of replication of nucleic acids, studies of permeability of films to substances, and so on. Particularly, the method of the invention is effective for use in luminescence immunoassays in which an antigen-antibody reaction is utilized.

**Claims**

1. A method of analyzing a chemiluminescent substance comprising:

placing a working electrode, a counter electrode and a reference electrode in a liquid sample containing an electrochemiluminescent substance capable of chemiluminescence through electrolytic oxidation in the presence of activated oxygen;

setting the working electrode at an oxidation potential for the electrochemiluminescent substance in the presence of activated oxygen to thereby cause electrochemiluminescence at the working electrode; and

measuring the amount of the electrochemiluminescence,

wherein the potential of the working electrode is controlled by use of the reference electrode to below a potential at which gaseous oxygen is generated through electrolysis of water.

2. The method according to claim 1, wherein the liquid sample has a pH of from 5 to 9.
A determination method wherein a reference electrode disposed in the aqueous solution is used to thereby control the potential of the anode to below a potential necessary for generation of gaseous oxygen through electrolysis of water.

3. The method according to claim 1, wherein the set potential of the working electrode is not lower than a potential at which oxygen is generated.

4. The method according to claim 1, wherein the set potential of the working electrode is below the potential at which gaseous oxygen is generated through electrolysis of water and within the range of 100 mV from the gas generation potential.

5. The method according to claim 1, wherein the electrochemiluminescent substance is luminol, luciferin or an electrochemiluminescent derivative thereof.

6. An apparatus for measurement of electrochemiluminescence comprising a vessel having two plates disposed opposite to each other to constitute vessel walls, a working electrode, a counter electrode, and a reference electrode, said working, counter and reference electrodes provided at an inner surface of one of said plates, and the other of said plates being light transmissive at least at its portion opposed to said working electrode.

7. An apparatus for measurement of electrochemiluminescence comprising a vessel having two plates disposed opposite to each other to constitute vessel walls, a working electrode, a reference electrode, and a counter electrode comprising a light transmitting material, said working and reference electrodes provided at an inner surface of one of said plates, and said counter electrode provided at least at that portion of the other of said plates which is opposed to said working electrode.

# F I G. 1

# F I G. 2

# F I G. 3

# F I G.4

# F I G. 5

# INTERNATIONAL SEARCH REPORT

International Application No    PCT/JP92/00173

**I. CLASSIFICATION OF SUBJECT MATTER** (if several classification symbols apply, indicate all) [6]

According to International Patent Classification (IPC) or to both National Classification and IPC

Int. Cl$^5$    G01N21/76, G01N33/536

**II. FIELDS SEARCHED**

| Minimum Documentation Searched [7] | |
|---|---|
| Classification System | Classification Symbols |
| IPC | G01N21/76, G01N33/536 |

| Documentation Searched other than Minimum Documentation to the Extent that such Documents are Included in the Fields Searched [8] |
|---|
| Jitsuyo Shinan Koho                1926 - 1991 |
| Kokai Jitsuyo Shinan Koho      1971 - 1991 |

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| P | JP, A, 3-107746 (TDK Corp.), May 8, 1991 (08. 05. 91), (Family: none) | 1-7 |
| A | JP, A, 1-153940 (TDK Corp.), June 16, 1989 (16. 06. 89), (Family: none) | 1-7 |

\* Special categories of cited documents: [10]

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance: the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

**IV. CERTIFICATION**

| Date of the Actual Completion of the International Search | Date of Mailing of this International Search Report |
|---|---|
| May 11, 1992 (11. 05. 92) | June 2, 1992 (02. 06. 92) |

| International Searching Authority | Signature of Authorized Officer |
|---|---|
| Japanese Patent Office | |